# EUROPÄISCHE PATENTANMELDUNG

(11) **EP 1 932 821 A1**
(43) Veröffentlichungstag der Anmeldung: **18.06.2008**
(21) Anmeldenummer: 07121645.1
(22) Anmeldetag: 27.11.2007
(51) Int. Cl.: C07C 29/50, C07C 35/08, C07C 45/33, C07C 49/303

(54) **Verfahren zur Herstellung von Oxidationsprodukten des Cyclohexans**

(30) Priorität: 12.12.2006 EP 06125873
(71) Anmelder: BASF SE, 67056 Ludwigshafen (DE)
(72) Erfinder: Rehfinger, Alwin, 67112 Mutterstadt (DE); Böhling, Ralf, 64653 Lorsch (DE); Mattke, Torsten, 67251 Freinsheim (DE); Schimpf, Axel, 69121 Heidelberg (DE)

(57) **Zusammenfassung**

Verfahren zur Herstellung von Oxidationsprodukten des Cyclohexans durch Oxidation von flüssigem Cyclohexan mit sauerstoffhaltigen Gasen bei erhöhter Temperatur und unter erhöhtem Druck, dadurch gekennzeichnet, dass die Oxidation in einer mikrostrukturierten Reaktionszone mit parallelen Kanälen einer spezifischen Wärmeaustauschoberfläche von insgesamt mehr als 1600 m²/m³ durchgeführt wird, wobei das flüssige Cyclohexan und die sauerstoffhaltigen Gase im Volumenstromverhältnis von 0,05 bis 20 im Gleichstrom unter einem Druck von 5 bis 150 bar in und durch die Reaktionszone gefördert und bei einer Temperatur von 150 bis 300°C umgesetzt und die Oxidationsprodukte gekühlt und ausgetragen werden.

## Beschreibung

Die Erfindung betrifft ein Verfahren zur Herstellung von Oxidationsprodukten des Cyclohexans durch Oxidation von flüssigem Cyclohexan mit sauerstoffhaltigen Gasen bei erhöhter Temperatur und unter erhöhtem Druck.

Die Oxidation von Kohlenwasserstoffen mit molekularem Sauerstoff und insbesondere die Oxidation von Cyclohexan zu Cyclohexanol und Cyclohexanon ist nach verschiedenen Verfahren möglich. Ein Problem dabei ist, dass die gewünschten Wertprodukte ihrerseits ebenfalls oxidiert werden können unter Erhalt unerwünschter Nebenprodukte oder letztendlich Kohlendioxid und Wasser. Dies führt nachteiligerweise zu einer Verminderung der Selektivität der Oxidationsreaktion.

Als eine technisch bedeutsame Oxidation wird in Weissermel/Arpe, Industrielle Organische Chemie, 4. Auflage, VCH Weinheim, 1994, Seite 260 ff die Oxidation von Cyclohexan zu einem Gemisch enthaltend Cyclohexanol und Cyclohexanon in der Flüssigphase mit Luft in Gegenwart von Mangan- oder Cobalt-Salzen als Katalysator bei 125-165°C und einem Druck im Bereich von 8 bis 15 bar (absolut) beschrieben.

Der Cyclohexan-Umsatz wird dabei begrenzt, um eine technisch sinnvolle Selektivität zu erzielen. Gemäß Arpentier et al., The Technology of Catalytic Oxidations, Editions Technip 2001, Seite 226 ff beträgt die Selektivität bei Cyclohexan-Umsätzen im Bereich von 1-2% ca. 90%, während sie bereits bei Umsätzen von 4-5% auf 77-85% abfällt.

Das nicht umgesetzte Cyclohexan muss in einer nachgeschalteten Destillationskolonne aufgearbeitet und in die Oxidationsstufe zurückgeführt werden.

Weiterhin ist aus der DE-PS 1 287 575 ein Verfahren zur Herstellung von Teiloxidationsprodukten des Cyclohexans bekannt, bei dem Cyclohexan von oben in eine mehrstufige Reaktionszone zugeleitet wird und molekularen Sauerstoff enthaltende Gas im Gegenstrom geführt werden. Die Oxidation wird bei erhöhter Temperatur und unter erhöhtem Druck durchgeführt, wobei die Geschwindigkeit der Gaszuführung der Geschwindigkeit des Sauerstoffverbrauchs in jeder Stufe im wesentlichen entsprechen soll und in die letzte Oxidationsstufe zusätzlich Inertgas zugeführt wird. Eine solche Arbeitsweise erlaubt indes keine gleichmäßige Zuführung von molekularem Sauerstoff und führt insbesondere zu einem ungleichmäßigen Fließen des Reaktionsgemisches. Darüber hinaus kann es zur Ausbildung von Sauerstoff enthaltenden Gaspuffern kommen, was wegen der Explosionsgefahr nicht unproblematisch ist und erheblichen sicherheitstechnischen Aufwand erforderlich macht.

Die EP-A-0 135 718 beschreibt ein Verfahren zur kontinuierlichen Oxidation von Kohlenwasserstoffen in flüssiger Phase, insbesondere zur Oxidation von Cyclohexan, bei dem ein sauerstoffhaltiges Gas an mehreren Stellen einer Reaktionszone über nach unten gerichtete Düsenöffnungen in das flüssige Reaktionsgemisch eingeleitet wird. Die Reaktionszone umfasst mehrere Kammern, so dass sich keine zusammenhängende Gasphase ausbilden kann. Es wird durch eine senkrecht stehende, mit Hilfe von Lochblechen in Kammern unterteilte Blasensäule von unten nach oben Cyclohexan mit einem darin gelösten Kobaltkatalysator gefördert. Oberhalb der Lochbleche wird über Düsen das sauerstoffhaltige Gas eingespeist, wobei durch eine genau definierte Gasaustrittsgeschwindigkeit und Gasmenge Blasen von definierter Größe entstehen. Das Verfahren gewährleistet zwar, dass das flüssige Reaktionsgemisch über das Volumen der Reaktionszone im wesentlichen gleichmäßig mit molekularem Sauerstoff in Berührung gebracht wird, es befriedigt jedoch nicht in jeder Hinsicht, sei es, dass die Selektivität der Oxidation bezüglich der Bildung von Cyclohexanol und Cyclohexanon nicht ausreichend ist, oder sei es, dass die Raumzeitausbeute begrenzt ist.

Es war daher Aufgabe der Erfindung, ein verbessertes Verfahren zur Herstellung von Oxidationsprodukten des Cyclohexans zu schaffen, mit dem sich das Potential möglichst hoher Reaktionsgeschwindigkeiten bei Aufrechterhaltung einer hochselektiven Oxidation mit minimalen sicherheitstechnischen Anforderungen realisieren lässt.

Zur Lösung dieser Aufgabe werden die im Kennzeichen des Anspruchs 1 enthaltenen Maßnahmen vorgeschlagen.

Erfindungsgemäß wird die Oxidation von flüssigem Cyclohexan mit Gasen in einer mikrostrukturierten Reaktionszone durchgeführt. Unter einer mikrostrukturierten Reaktionszone im Sinne der Erfindung werden Zonen eines Reaktors mit parallelen Kanälen mit hydraulischen Kanaldurchmessern von weniger als 2,5 mm, insbesondere weniger als 1 mm und einer spezifischen inneren Oberfläche von insgesamt mehr als 1600 m²/m³, vorzugsweise mehr als 4000 m²/m³ verstanden. Eine solche Reaktionszone wird im Allgemeinen aus modular zusammengesetzten Bauteilen gebildet, wobei die einzelnen Bauteile an jeweils übereinstimmenden Stellen Bohrungen oder Vertiefungen aufweisen, die in Gebrauchsstellung Kanäle für die Stoffe-, Reaktions- und Wärmeführung bilden. Die Kanäle können sowohl vertikal als auch horizontal ausgerichtet sein. Mikrostrukturierte Reaktoren zeichnen sich durch eine sehr gute Wärmeübertragungsleistung verbunden mit einem sehr schnellen Stofftransport aus.

Die Oxidation des Cyclohexans in der Flüssigphase wird mittels Gasen, die molekularen Sauerstoff enthalten, durchgeführt. Eine Sauerstoffkonzentration im Bereich von 5 bis 30 Vol.-% hat sich insgesamt als zweckmäßig erwiesen. Die Vermischung der Gasphase mit der Flüssigphase kann außerhalb und/oder innerhalb der mikrostrukturierten Reaktionszone erfolgen. Bei der Vermischung außerhalb der Reaktionszone wird dieser ein geeignetes Mischorgan, z.B. eine Mischpumpe oder ein Statikmischer, vorgeschaltet. Hierbei kann die Gasphase in Abhängigkeit des Volumenstromverhältnisses von Cyclohexan und Gas sowie von Druck und Temperatur auch bereits vollständig in der Flüssigphase gelöst werden. Vorzugsweise erfolgt die Vermischung jedoch innerhalb der Reaktionszone durch Injektionsvermischung, wobei entsprechend der Löslichkeit der sauerstoffhaltigen Gase in dem flüssigen Cyclohexan durch Abstimmung der Prozessparameter Druck und Temperatur in der Reaktionszone sowie der Verweilzeit des Gemischs in der Reaktionszone der Sauerstoff praktisch vollständig umgesetzt wird. Für einen guten Stoffaustausch verbunden mit rückvermischungsarmer Durchführung des Verfahrens hat sich ein Volumenstromverhältnis von Gas- zu Flüssigphase von 0.05 bis 20, zweckmäßig 0,1 bis 10, insbesondere 0,2 bis 5 als vorteilhaft gezeigt.

Erfindungsgemäß werden die sauerstoffhaltigen Gase und das flüssige Cyclohexan im Gleichstrom unter einem Druck von 5 bis 150 bar, bevorzugt 35 bis 100 bar in und durch die mikrostrukturierte Reaktionszone gefördert, gegebenenfalls mehrfach in Teilströme aufgeteilt und die Teilströme wieder zusammengeführt. Die Oxidation des Cyclohexans erfolgt bei einer Temperatur von 150 bis 300°C, vorzugsweise 200 bis 270°C, insbesondere 220 bis 260°C.

Die Verweilzeit des Reaktionsgemisches in der Reaktionszone liegt allgemein zwischen 1 s und 2000 s, bevorzugt zwischen 2 und 900 s und besonders bevorzugt zwischen 3 und 60 s.

Für eine selektive Reaktionsführung der Oxidationsreaktion hat sich erfindungsgemäß eine Mikrostruktur mit zwei unabhängig voneinander regelbaren Temperaturzonen als besonders vorteilhaft erwiesen, wobei die Temperatur der Oxidationsprodukte in der zweiten Zone, der sogenannten Quenchzone, um mindestens 10°C bevorzugt um mindestens 20°C gemindert wird.

Nach einem weiteren Merkmal der Erfindung wird die Oxidation in Gegenwart eines Katalysators durchgeführt. Es können homogene oder heterogene Katalysatoren verwendet werden.

Setzt man einen homogenen Katalysator ein, so kann dieser dem zu oxidierenden Cyclohexan zugegeben werden und zusammen mit den flüssigen Oxidationsprodukten wieder aus der Reaktionszone ausgetragen werden. Hierbei kann sich auf der inneren Mantelfläche der Kanäle ein katalytisch wirksamer Belag ausbilden und damit insitu eine zusätzliche katalytische Wirkung entfalten. Setzt man einen heterogenen Katalysator ein, so kann dieser nach an sich bekannten Verfahren in der Reaktionszone fixiert werden.

Im allgemeinen können für die Herstellung von Cyclohexanol, Cyclohexanon oder deren Gemische die an sich bekannten Katalysatoren, insbesondere Cobalt- oder Manganhaltige Salze eingesetzt werden.

Gemäß einer speziellen Ausführungsform des Verfahrens wird die Oxidation in Kanälen mit einer katalytisch wirksamen Beschichtung, vorzugsweise mit einer inerten Siliziumbeschichtung, wie dies im Unteranspruch 4 angegeben ist, durchgeführt. Hierdurch gelingt es unselektive Wandreaktionen zu unterdrücken.

Gegenüber dem Stand der Technik können nach der Erfindung sehr hohe Raumzeitausbeuten für die Summe aus Cyclohexanol, Cyclohexanon und Cyclohexylhydroperoxid, gerechnet als Cyclohexanol, von über 0,5 bis zu etwa 8 t pro m³ Reaktionsvolumen (Gas- und Flüssigkeitsvolumen) und h erzielt werden. Die Selektivität zu den genannten Wert- bzw. Zwischenprodukten beträgt dabei in Summe bis zu 89 %. Die mikrostrukturierte Reaktionszone ist störungsunanfällig und kann besonders kleinbauend ausgeführt sein. Der sicherheitsrelevante Holdup an heißem Cyclohexan lässt sich auf einen sehr geringen Anteil reduzieren. Die mikrostrukturierte Reaktionszone kann wegen der geringen Kanalabmessungen eigensicher gegen mögliche detonativ verlaufende Reaktionen in der Gasphase gebaut werden.

Cyclohexanol und Cyclohexanon sind Ausgangsprodukte zur Herstellung von Faserrohstoffen wie Dicarbonsäuren oder Lactamen.

Das Verfahren der Erfindung wird durch folgende Beispiele erläutert:

### Beispiel 1

Einer mikrostrukturierten Reaktionszone, bestehend aus vier parallelen und symmetrisch zueinander angeordneten und mit einer Silizium-Passivierungsschicht versehenen Kanälen einer Länge von 2,7 m und einer lichten Weite von 0,25 mm wurden 92,5 g/h Cyclohexan und 8,64 Nl/h Luft bei einem Druck von 60 bar zugeführt. Die Luft wurde auf vier getrennte, zur Druckverlusterzeugung jeweils 3 m lange Kapillarleitungen mit 0,1 mm Innendurchmesser zu gleichen Teilen aufgeteilt und dann über diese direkt in die einzelnen Kanäle der Reaktionszone injiziert. Eine besondere Verteilung des zugeführten Cyclohexans erfolgte nicht. Sowohl Cyclohexan als auch die Luft wurden vor der Reaktionszone auf 150°C vorgeheizt.

Das Komponentengemisch wurde in den Kanälen über einen außen im Gleichstrom anliegenden Ölkreislauf auf eine Temperatur von 252,5°C erwärmt und bei 60 bar durch die Reaktionszone gefördert. Die Öltemperatur betrug am Eingang der Reaktionszone 256°C und am Ausgang 250°C. Die rechnerische Verweilzeit des Komponentengemisches in der Reaktionszone betrug ohne Berücksichtigung seines Gasanteils 9,4 s.
Das Komponentengemisch wurde noch in der Reaktionszone in der sich anschließenden Quenchzone einer Länge von 0,3 m in den Kanälen der Mikrostruktur auf 150°C abgekühlt, dann die 4 Teilströme zusammengeführt, zur Homogenisierung mit Isopropanol versetzt und auf 25°C abgekühlt. Anschließend wurde zur Inertisierung Stickstoff zudosiert und die Gasphase von der Flüssigphase in einem Abscheidegefäß unter Druck getrennt. Die Oxidationsprodukte wurden danach auf Umgebungsdruck entspannt.

Nach dieser Fahrweise wurde das zudosierte Cyclohexan zu 5,1 % umgesetzt. Die Selektivität zu Cyclohexanol, Cyclohexanon und Cyclohexylhydroperoxid betrug in Summe 77 %. Die erzielte Raum-Zeit-Ausbeute lag unter Berücksichtigung der genannten Wertprodukte als Cyclohexanol bei 8,4 t Cyclohexanol/m³h.

### Beispiel 2

Wie in Beispiel 1 beschrieben, wurden 397 g/h Cyclohexan und 13,0 Nl/h Luft einer mikrostrukturierten Reaktionszone bei einem Druck von 40 bar zugeführt. Die Reaktionszone bestand aus vier 1,01 m langen Kanälen mit einer lichten Weite von 2,16 mm, die auf ihren inneren Oberflächen mit einer Siliziumbeschichtung zur Oberflächenpassivierung versehen waren. Die Verweilzeit des Komponentengemisches mit einem Gasanteil von etwa 45 Vol-% in der Reaktionszone betrug 40 s bei einer mittleren Temperatur von 223°C, wobei die Öltemperatur am Eingang auf 227°C und die Öltemperatur am Ausgang auf 219°C eingestellt war. In der sich anschließenden 0,3 m langen Quenchzone wurde das Komponentengemisch auf 150°C abgekühlt und wie zuvor beschrieben weiterbehandelt.

Es wurde nach diesen Maßnahmen Cyclohexan zu 2,1 % umgesetzt. Die Selektivität zu Cyclohexanol, Cyclohexanon und Cyclohexylhydroperoxid betrug in Summe 89 %. Die erzielte Raum-Zeit-Ausbeute lag bei 0,58 t Cyclohexanol/m³h.

## Patentansprüche

1. Verfahren zur Herstellung von Oxidationsprodukten des Cyclohexans durch Oxidation von flüssigem Cyclohexan mit sauerstoffhaltigen Gasen bei erhöhter Temperatur und unter erhöhtem Druck, **dadurch gekennzeichnet, dass** die Oxidation in einer mikrostrukturierten Reaktionszone mit parallelen Kanälen einer spezifischen inneren Oberfläche von insgesamt mehr als 1600 m²/m³ durchgeführt wird, wobei das flüssige Cyclohexan und die sauerstoffhaltigen Gase im Volumenstromverhältnis von 0,05 bis 20 im Gleichstrom unter einem Druck von 5 bis 150 bar in und durch die Reaktionszone gefördert und bei einer Temperatur von 150 bis 300°C umgesetzt und die Oxidationsprodukte gekühlt und ausgetragen werden.

2. Verfahren nach Anspruch 1, **dadurch gekennzeichnet, dass** die Oxidation in Gegenwart eines Katalysators durchgeführt wird.

3. Verfahren nach den Ansprüchen 1 und 2, **dadurch gekennzeichnet, dass** die Oxidation in Kanälen mit einer katalytisch wirksamen Beschichtung der inneren Oberflächen durchgeführt wird.

4. Verfahren nach den Ansprüchen 1 bis 3, **dadurch gekennzeichnet, dass** die Oxidation in Kanälen mit einer inerten Siliziumbeschichtung durchgeführt wird.

5. Verfahren nach Anspruch 1, **dadurch gekennzeichnet, dass** das flüssige Cyclohexan und die sauerstoffhaltigen Gase im Volumenstromverhältnis von 0,2 bis 5 in die Reaktionszone gefördert werden.

6. Verfahren nach Anspruch 1, **dadurch gekennzeichnet, dass** die Temperatur der umgesetzten Oxidationsprodukte in der Reaktionszone um mindestens 10°C reduziert wird.
